# EUROPEAN PATENT APPLICATION

(11) **EP 1 502 611 A2**
(43) Date of publication of application: **02.02.2005**
(21) Application number: 04076557.0
(22) Date of filing: 27.05.2004
(51) Int. Cl.: A61M 1/36, A61M 25/06

(54) **Sheath with air trap**

(30) Priority: 29.07.2003 US 629530
(71) Applicant: Cryocor, Inc., San Diego, California 92121 (US)
(72) Inventor: Castellano, Thomas, Temecula California 92591 (US); Lentz, David J., La Jolla California 92037 (US)
(74) Representative: Shortt, Peter Bernard

(57) **Abstract**

A system for facilitating the insertion of a medical device into a patient, comprises a flexible sheath and an air trap chamber coupled thereto. The air trap chamber includes a device insertion opening and an exit opening which allow the distal end of a medical device to pass through the chamber before it enters the sheath. The chamber also includes a gas removal port opening for preventing air from entering the sheath by removing air from the chamber.

## Description

### FIELD OF THE INVENTION

The present invention relates to devices and methods that facilitate the insertion of devices into body lumens. In particular, the present invention relates to devices that reduce the introduction of emboli during insertion of cardiac devices into blood vessels.

### BACKGROUND OF THE INVENTION

During certain cardiac procedures, catheters or other devices may be inserted into a patient's vascular system and pushed through blood vessels to reach a desired location. Once the desired location has been reached, the tissue at that location may be treated using any of a variety of devices. For example, treatment of certain cardiac arrhythmias which occur when contraction initiating signals originate within one or more of the pulmonary veins rather than at the sino-atrial node (SA) may include the introduction of a catheter into the left atrium of the patient to form a conduction block between the source of the improper contraction initiating signals and the left atrium.

In many such procedures, cardiac sheaths are used to facilitate insertion and exchange of the devices used to treat the affected tissue. These cardiac sheaths are tubes that are inserted into patients' vascular systems to act as guides for the other devices. For example, the distal end of a cardiac sheath may be inserted into a patient's femoral vein and advanced to the site to be treated with an open proximal end thereof remaining accessible from outside the patient. A catheter or other device may then be inserted through the sheath, which guides the device into the vascular system. If a first device needs to be replaced with a second, the first device is withdrawn from the sheath and the second device is inserted therethrough.

When a device is inserted through the sheath, air may be carried into the sheath with the device. This air may form bubbles, or emboli, when entering the blood stream, preventing normal blood flow to the heart and brain and potentially causing tissue damage or death of the patient. In particular, if devices used in treating the patient must be exchanged repeatedly via a sheath, great care must be exercised to prevent formation of emboli. Furthermore, the leakage of blood from such a sheath must be prevented while allowing insertion and retraction of devices therethrough.

### SUMMARY OF THE INVENTION

The present invention is directed to a system for facilitating the insertion of a medical device into a patient. Specifically, the system includes a flexible sheath which, when in an operative position, is received within a body lumen in the vasculature of the patient. An air trap chamber is coupled to the extracorporeal proximal end of the flexible sheath, and the air trap chamber includes a device insertion opening through which the distal end of a device may be inserted into the air trap chamber. The air trap chamber also includes a device exit opening through which the distal end of the device may pass out of the air trap chamber and into the flexible sheath. Further, there is a gas removal port opening that is formed through a surface of the air trap chamber. In operation, the gas removal port functions in combination with a proximal hemostasis valve (disposed at the device insertion opening of the air trap chamber) and a distal hemostasis valve (disposed at the device exit opening of the air trap chamber) to keep air from entering the sheath as the device is inserted into the patient's vasculature.

A system for preventing the introduction of air into a patient during the insertion of a medical device into the vasculature of the patient Includes a flexible sheath that has a proximal end and a distal end and is formed with a lumen. During use of the system, the sheath is prepositioned in the vasculature of a patient to extracorporeally expose the proximal end of the sheath.

The system of the present invention also includes an air trap chamber that is formed with an insertion opening, an exit opening and a gas removal port. Preferably, the insertion opening of the chamber is covered by a proximal hemostasis valve, and its exit opening is covered by a distal hemostasis valve. As intended for the present invention, the exit opening is engageable with the extracorporeal end of the sheath. With this engagement, a pathway is established for advancement of the medical device into the vasculature of the patient. Specifically, this pathway is established sequentially through the insertion opening, through the air trap chamber, through the lumen of the sheath, and into the vasculature.

An important aspect of the present invention is that it provides for the removal of air from the air trap chamber during the advancement of a medical device through the system. To do this the air trap chamber is formed with a top portion that is shaped to trap and direct gas bubbles toward the gas removal port.

For one embodiment of the present invention, the removal of air is accomplished using a syringe that is connected in fluid communication with the gas removal port of the air trap chamber. In an alternate embodiment of the present invention, the gas removal function is accomplished using a gas separator that is connected to the air trap chamber through a return tube. This alternate embodiment also includes a fluid pump that is connected in fluid communication between the gas removal port of the air trap chamber, and the gas separator. The fluid pump, which may be a peristaltic type pump, is used to pump fluid from the air trap chamber, and into the gas separator. The gas separator then removes gas from the fluid before returning it through the return tube to the air trap chamber.

Additionally, the air trap chamber can have a second gas removal port for venting gas from the air trap chamber whenever the air trap chamber is being filled with a fluid, such as a saline solution. Further, the system of the present invention can include an adapter(s) for connecting a different sized sheath(s) to the exit opening of the air trap chamber.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a side elevation view showing an exemplary embodiment of the sheath according to the present invention; and
Figure 2 is a side elevation view of a second exemplary embodiment of the sheath according to the present invention.

### DESCRIPTION OF THE PREFERRED EMBODIMENTS

The present invention may be further understood with reference to the following description and the appended drawings, wherein like elements are referred to with the same reference numerals.

As described above, using cardiac sheaths to alleviate the problems associated with repeated insertion and removal of devices may also increase the possibility that air emboli may be introduced in the bloodstream. These gas bubbles are generally caused by air introduced into the cardiac sheath when a catheter or other device is inserted thereinto. Although catheters with large diameters may be more prone to introduce air into the sheath, any device inserted thereinto may potentially introduce air, making these procedures difficult and time consuming. The exemplary embodiments of the sheath according to the present invention prevent formation of emboli, by providing a mechanism for removing bubbles of gas from the cardiac sheath before they can enter the bloodstream. The present invention also reduces reliance on the surgeon's technique in preventing introduction of air, and makes the insertion procedure faster.

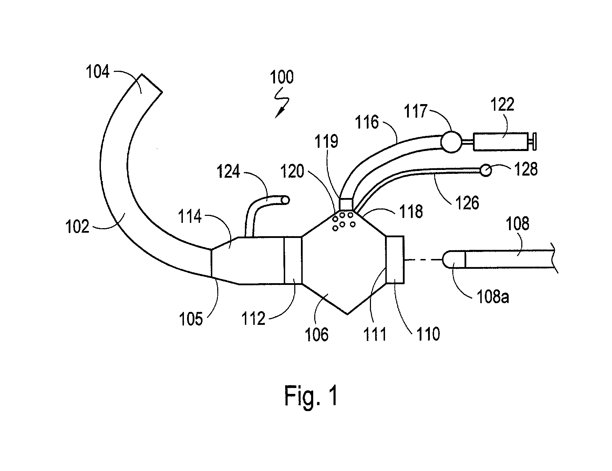

Figure 1 shows an exemplary embodiment of a sheath 100 according to the present invention. The cardiac sheath 100 may be used, for example, to assist in inserting catheters and other devices into the vasculature of a patient. Sheath 100 includes a tubular body 102 designed to be inserted into the selected vessel of the patient. The tubular body 102 may have, for example, a length of between 10 cm and 100 cm, and a diameter of between 3 and 34 French depending on the particular use for which it is intended. The tubular body 102 has a distal end 104 adapted for insertion into a vessel of the patient, and a proximal end 105 that remains outside of the patient's body. Once inserted into a blood vessel, the tubular body 102 of sheath 100 is purged of air using techniques that are well known in the pertinent art.

An air trap chamber 106 is connected to the proximal end 105 of tubular body 102 to permit insertion of a device 108 into the sheath 100. The device 108 may be a catheter or other type of interventional device, which is inserted into the sheath 100 and which may then be further advanced through the sheath 100 to exit from the distal end 104. The device 108 will then enter the blood vessel, heart chamber or other targeted body cavity until a distal portion 108a of the device 108 reaches a desired position. For example, in a procedure to treat cardiac arrhythmia originating in a pulmonary vein, the sheath 100 is advanced into the patient's right atrium via the inferior vena cava and then passed through to the left atrium via the Seldinger technique as is known in the art. The device 108 may be pushed through the sheath 100 until the distal end 108a exits the distal end 104 to protrude into the left atrium. An adapter 114 may then be placed between the air trap chamber 106 and the tubular body 102, to help guide the distal end 108a of the device 108 into the sheath 100. The adapter 114 may alternatively be part of the air trap chamber 106, or may be formed at a proximal end of tubular body 102.

A first side of the air trap chamber 106 is in fluid connection with the patient's vascular system, through tubular body 102 and on a second side thereof is exposed to the outside environment. The sheath 100 must therefore prevent loss of blood through the air trap chamber 106. In the exemplary embodiment shown in Figure 1, a proximal hemostasis valve 110 is placed at a device insertion opening 111 of the air trap chamber 106. The hemostasis valve 110, or an adjustable hemostasis valve, is used to control flow through the device insertion opening when the device 108 is inserted, as well as when the device 108 is not present. In this embodiment, the air trap chamber 106 may be partially filled with blood after the sheath 100 has been inserted into a blood vessel, and the proximal hemostasis valve 110 prevents the fluid from leaking from the air trap chamber 106 out of the body.

When a device 108 is inserted into a cardiac sheath 100, the proximal hemostasis valve 110 is opened sufficiently to allow passage of the device 108 through the valve 110, but it retains a seal around the outer surface of the device 108 so that blood or saline does not leak out. After device 108 has entered into the air trap chamber 106, it is still separated from the tubular body 102, and thus from the patient's blood vessel by the distal hemostasis valve 112. After the device 108 has been pushed through the proximal hemostasis valve 110, it is further pushed through the distal hemostasis valve 112, to enter the tubular body 102 and pass from there into the blood vessel of the patient. To do this, the distal hemostasis valve 112 opens enough to allow the device 108 to pass through but it also forms a seal around the device 108 to restrict the flow of blood between the tubular body 102 and the air trap chamber 106. The distal valve 112 also restricts flow between the air trap chamber 106 and the patient's vascular system.

As the device 108 is introduced into the cardiac sheath 100, air may also be introduced thereinto as the proximal hemostasis valve 110 opens. The air trap chamber 106 traps this air and facilitates its removal from the sheath 100 before it can pass through the distal hemostasis valve 112 and enter the tubular body 102. In one exemplary embodiment, the air trap chamber 106 may have a sloping upper portion 118 that converges to a gas removal port 119. Air bubbles 120 which tend to move upward due to their buoyancy collect at the highest point of the air trap chamber 106. The shape of the upper portion 118 takes advantage of this buoyancy and traps the bubbles 120 near the port 119. A gas extractor 122 may then be used to remove the bubbles 120 from the air trap chamber 106 through the gas removal port 119. This may be done either directly, or through a tube 116 with a stopcock/valve 117. In one exemplary embodiment, the extractor 122 may be formed as a syringe or other vacuum generating device as would be understood by those of skill in the art. In another exemplary embodiment of the invention, a second gas removal port 124 may be added to sheath 100, to remove gas bubbles that may collect in the adapter 114. Further, yet another gas removal port 126, with a stopcock/valve 128, can be connected to chamber 106 for venting purposes as chamber 106 is filling with fluid prior to the use of the present invention.

An exemplary method of utilizing the sheath 100 according to the embodiment shown in Figure 1 is described below. When it is necessary to insert a device 108 into the sheath 100, the distal end 108a of the device 108 is pushed against the proximal hemostasis valve 110. This opens the valve 110 sufficiently to allow the device 108 to enter the air trap chamber 106. The valve 110 also prevents a back flow of fluid therefrom. Since air may be introduced in chamber 106 together with the device 108 at this time, suction or a vacuum is applied through port 119 to remove any gas bubbles 120 that may have collected in the top part 118 of the air trap chamber 106. Once the trapped gas has been removed, the device 108 may then be pushed the rest of the way through the distal hemostasis valve 112 and the tubular body 102 until the distal end 108a extends into the patient's blood vessel.

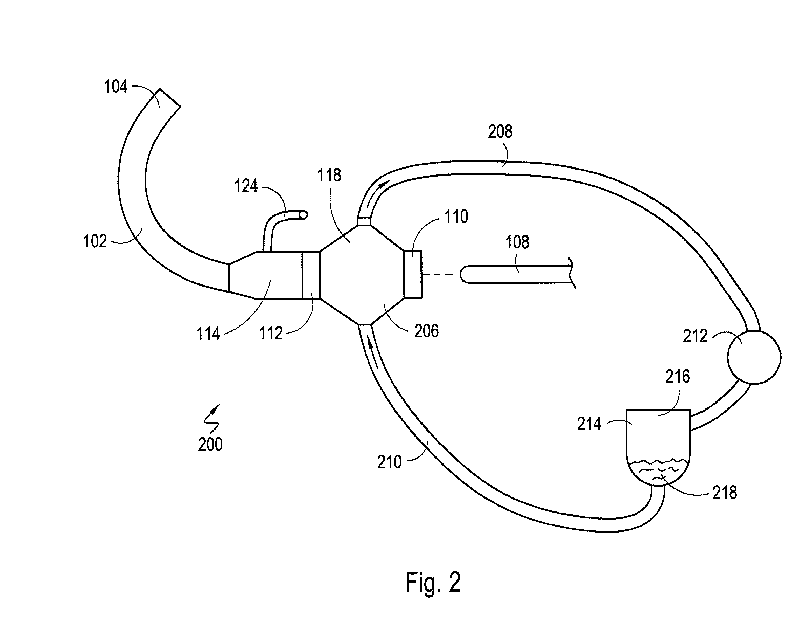

Figure 2 shows a sheath 200 according to another exemplary embodiment of the present invention. In this embodiment, the tubular body 102 is similar to the one described above, with a distal end 104 that is introduced into a patient's blood vessel and with proximal and distal hemostasis valves 112 and 110 allowing introduction of the device 108 thereinto while restricting the flow of fluids therefrom. The air trap chamber 206, however, is designed not to collect air bubbles in a specific location for later removal but to continuously remove gas bubbles therefrom.

In the cardiac sheath 200 shown in Figure 2, an outlet tube 208 connects the air trap chamber 206 to a gas separator 214. For example, to remove the gas, the outlet tube 208 may be connected to the top portion 118 of the chamber 206, where gas tends to accumulate. A return tube 210 may be connected to a bottom portion of the air trap chamber 206, to circulate fluid that has been treated by gas separator 214. In one exemplary embodiment, the gas separator 214 comprises a bottom portion 218 in which liquids are retained, and a top portion 216 where buoyant gases tend to accumulate. The inlet tube 208 may be connected to the top portion 216 so that gas bubbles present in the fluid are separated therefrom with the gases remaining in the top portion 216. The return line 210 may be connected to the bottom portion 218 to ensure that only liquid is carried back to the air trap chamber 206. It will be apparent to one skilled in the art that any conventional gas separator design may be used according to the invention.

Optionally, a pump 212 may be included along the outlet tube 208 or the return tube 210. The pump 212 ensures that a continuous flow of liquid passes through the air trap chamber 206, removing any air bubbles that might otherwise be introduced by the device 108. In one embodiment, the pump 212 is a peristaltic pump, so that it will not introduce air into the system. However, other types of pumps may also be used according to the invention. The fluid that flows through the air trap chamber 206 may be, for example, a saline solution that is harmless if it is introduced into the vascular system during insertion of the device 108.

According to the embodiment shown in Figure 2. device 108 may be inserted directly through both proximal and distal hemostasis valves 110, 112 and into the tubular body 102, without having to carry out a separate gas removal step. Since fluid flows continuously through the air trap chamber 206, any air introduced is promptly removed by the flowing fluid, and is carried to the gas separator 214. A constant flow of liquid, which may be a saline solution, without gas bubbles is provided to the air trap chamber 206. Thus, the cardiac sheath 200 makes the task of inserting and exchanging devices 108 rapid and convenient.

In the preceding specification, the present invention has been described with reference to specific exemplary embodiments thereof. It will, however, be evident that various modifications and changes may be made thereto without departing from the broadest spirit and scope of the present invention as set forth in the claims that follow. The specification and drawings are accordingly to be regarded in an illustrative rather than restrictive sense. For example, while the invention has been described for use with left side cardiac procedures, it can be used with any surgical procedure where it must be ensured that air is not introduced into the patient's body.

While the particular Sheath with Air Trap as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A system for preventing the introduction of air into a patient during the insertion of a medical device into the vasculature thereof, the system comprising:
a flexible sheath formed with a lumen and positioned in the vasculature of the patient to expose an extracorporeal end thereof;
an air trap chamber having an insertion opening, an exit opening and a gas removal port, with the exit opening being engageable with the extracorporeal end of said sheath to establish a pathway for advancement of the device sequentially through the insertion opening, through the air trap chamber, through the lumen of said sheath, and into the vasculature; and
a means for removing air from said chamber through the gas removal port during an advancement of the device through said system.

2. A system as recited in claim 1 further comprising:
a proximal hemostasis valve disposed over the insertion opening of said air trap chamber; and
a distal hemostasis valve disposed over the device exit opening of said air trap chamber.

3. A system as recited in claim 1 wherein said gas removing means is a syringe.

4. A system as recited in claim 1 wherein said gas removing means comprises:
a gas separator connected in fluid communication with the air trap chamber through a return tube; and
a fluid pump connected in fluid communication between the gas removal port of said air trap chamber and said gas separator, for pumping fluid from said air trap chamber through said gas separator to remove gas from said fluid before returning the fluid through the return tube to said air trap chamber.

5. A system as recited in claim 4 wherein said fluid pump is a peristaltic pump.

6. A system as recited in claim 1 wherein said air trap chamber has a second gas removal port for venting gas from said air trap chamber as said air trap chamber is filled with a fluid.

7. A system as recited in claim 1 further comprising an adapter for connecting said sheath to the exit opening of said air trap chamber.

8. A system as recited in claim 1 wherein said air trap chamber has a top portion shaped to trap and direct gas bubbles toward the gas removal port.

9. A sheath to facilitate insertion of a medical device into a blood vessel, comprising:
a tubular body having a distal portion adapted to be inserted into a blood vessel;
an air trap chamber fluidly connected to a proximal portion of the tubular body;
a distal hemostasis valve separating the tubular body from the air trap chamber; and
a proximal hemostasis valve disposed at an opening of the air trap chamber for insertion of the device therethrough.

10. The sheath as recited in claim 9, wherein a longitudinal axis of the proximal hemostasis valve is aligned with a longitudinal axis of the distal hemostasis valve.

11. The sheath as recited in claim 9, further comprising a gas removal port formed through a surface of the air trap chamber.

12. The sheath as recited in claim 9, further comprising a gas separator chamber in fluid communication with the air trap chamber.

13. The sheath as recited in claim 12, further comprising a pump for circulating fluid between the air trap chamber and the gas separator chamber.

14. The sheath as recited in claim 9, wherein the air trap chamber has a portion shaped to direct gas bubbles to a collection portion of the air trap chamber.

15. A method for preventing the introduction of air into a patient during the insertion of a medical device into the vasculature thereof, the method comprising the steps of:
providing a flexible sheath formed with a lumen, the sheath being engageable with an air trap chamber having an insertion opening, an exit opening and a gas removal port,
positioning the sheath in the vasculature of a patient to expose an extracorporeal end thereof;
engaging the extracorporeal end of the sheath with the exit opening of the air trap chamber to establish a pathway for advancement of the medical device along a pathway sequentially through the insertion opening, through the air trap chamber, through the lumen of the sheath, and into the vasculature of the patient; and
removing air from said chamber through the gas removal port during an advancement of the device through the system.
